# EUROPÄISCHE PATENTSCHRIFT

(11) **EP 0 583 694 B1**
(45) Veröffentlichungstag und Bekanntmachung des Hinweises auf die Patenterteilung: **10.01.1996**
(21) Anmeldenummer: 93112549.6
(22) Anmeldetag: 05.08.1993
(51) Int. Cl.: C07C 255/19

(54) **Verfahren zur Herstellung von Cyanessigsäurealkylestern**
Process for the preparation of cyanoacetic acid alkylesters
Procédé de préparation d'alkylesters de l'acide cyanoacétique

(30) Priorität: 20.08.1992 DE 4227505
(43) Veröffentlichungstag der Anmeldung: 23.02.1994
(73) Patentinhaber: BASF Aktiengesellschaft, D-67063 Ludwigshafen (DE)
(72) Erfinder: Reichelt, Helmut, Dr., D-6730 Neustadt (DE); Grund, Clemens, Dr., D-6800 Mannheim 24 (DE)

(56) Entgegenhaltungen:
- WO-A-92/12962
- DE-A- 1 938 080
- US-A- 2 553 065
- J. FALBE 'Methoden der organischen Chemie (Houben-Weyl) Band E5' 1985 , GEORG THIEME VERLAG , STUTTGART, NEW YORK
- J. BARTELS ET AL 'Landolt-Börnstein, Zahlenwerte und Funktionen aus Physik-Chemie-Astronomie-Geophysik und Technik Band II' 1960, SPRINGER VERLAG, BERLIN-GÖTTINGEN-HEIDELBERG
- CHEMICAL ABSTRACTS, vol. 82, 1975, Columbus, Ohio, US; abstract no. 155242x,

## Beschreibung

Die vorliegende Erfindung betrifft ein neues Verfahren zur Herstellung von Cyanessigsäure-C₄-C₁₀-alkylestern durch Umsetzung von Cyanessigsäure mit C₄-C₁₀-Alkanolen.

Aus der DE-A-1 938 080 ist bekannt, Cyanessigsäure aus waßriger Lösung mittels n-Butanol zu extrahieren und den Extrakt in Gegenwart von Toluolsulfonsäure unter azeotroper Entfernung des Reaktionswassers in Cyanessigsäure-n-butylester umzuwandeln. Es hat sich jedoch gezeigt, daß bei dieser Verfahrensweise die Extraktion von Cyanessigsäure aus der wäßrigen Phase nur unvollständig gelingt.

Aufgabe der vorliegenden Erfindung war es daher, ein neues Verfahren zur Herstellung von Cyanessigsäure-C₄-C₁₀-alkylestern bereitzustellen, bei dem ebenfalls von einer wäßrigen Cyanessigsäurelösung ausgegangen wird, wobei aber auf die Extraktion der Cyanessigsäure verzichtet werden kann.

Es wurde nun gefunden, daß die Herstellung von Cyanessigsäure-C₄-C₁₀-alkylestern durch Umsetzung von Cyanessigsäure mit C₄-C₁₀-Alkanolen vorteilhaft gelingt, wenn man Cyanessigsäure in wäßrigem Medium mit der 5 bis 30 molaren Menge, bezogen auf 1 mol Cyanessigsäure, eines C₄-C₁₀-Alkanols bei einer Temperatur von 35 bis 150°C und bei einem Druck von 70 bis 1013 mbar in Gegenwart von katalytischen Mengen einer Säure umsetzt und während der Umsetzung ein C₄-C₁₀-Alkanol/Wasser-Azeotrop abtrennt.

Für das erfindungsgemäße Verfahren geeignete C₄-C₁₀-Alkanole sind z.B. Butanol, Isobutanol, sec-Butanol, tert-Butanol, Pentanol, Isopentanol, Neopentanol, tert-Pentanol, Hexanol, 2-Methylpentanol, Heptanol, 2-Methylhexanol, Octanol, Isooctanol, 2-Ethylhexanol, Nonanol, Isononanol, 2-Methyloctanol, Decanol, Isodecanol oder 2-Methylnonanol.

Die obigen Bezeichnungen Isooctanol, Isononanol und Isodecanol sind Trivialbezeichnungen und beziehen sich auf die in der Oxosynthese erhaltenen Alkohole - vgl. dazu Ullmanns Encyklopädie der technischen Chemie, 4. Auflage, Band 7, Seiten 215 bis 217, sowie Band 11, Seiten 435 und 436.

Bevorzugt ist eine Verfahrenweise zur Herstellung von Cyanessigsäure-C₄-C₈-alkylestern, wobei die Herstellung von Cyanessigsäure-C₄-alkylestern besonders hervorzuheben ist.

Im erfindungsgemäßen Verfahren wird die Veresterung von Cyanessigsäure in wäßrigem Medium vorgenommen. Dabei geht man in der Regel von einer wäßrigen Cyanessigsäurelösung aus, die einen Gehalt an Cyanessigsäure von 20 bis 70 Gew.-%, vorzugsweise 60 bis 70 Gew.-%, jeweils bezogen auf das Gewicht der Lösung, aufweist.

Je mol Cyanessigsäure kommen in der Regel 5 bis 30 mol, vorzugsweise 15 bis 20 mol, C₄-C₁₀-Alkanol zur Anwendung.

Erfindungsgemäß wird die Veresterung in Gegenwart von katalytischen Mengen einer Säure vorgenommen. Geeignete Säuren sind insbesondere starke bis mittelstarke anorganische oder organische Säuren, wie Schwefelsäure, Salzsäure, Phosphorsäure, Benzolsulfonsäure, o- oder p-Toluolsulfonsäure, Methansulfonsäure, Trifluormethansulfonsäure, Trifluoressigsäure oder Mono-, Di- oder Trichloressigsäure. Die Verwendung von Schwefelsäure oder p-Toluolsulfonsäure ist bevorzugt, wobei insbesondere die Verwendung von Schwefelsäure hervorzuheben ist.

Unter katalytischen Mengen werden üblicherweise 0,001 bis 0,01 mol Säure, bezogen auf 1 mol Cyanessigsäure verstanden.

Das erfindungsgemäße Verfahren wird bei einer Temperatur von 35 bis 150°C, vorzugsweise 50 bis 90°C, durchgeführt.

Der Druck im erfindungsgemäßen Verfahren beträgt 70 bis 1013 mbar, vorzugsweise 70 bis 270 mbar.

Das neue Verfahren wird zweckmäßig so durchgeführt, daß man in einer geeigneten Apparatur bei Raumtemperatur wäßrige Cyanessigsäurelösung, C₄-C₁₀-Alkanol und Säure (Katalysator) im obengenannten Mengenverhältnis vorlegt, auf die erfindungsgemäße Temperatur erhitzt, den erfindungsgemäßen Druck einstellt und gleichzeitig mit der Abtrennung das C₄-C₁₀-Alkanol/Wasser-Azeotrops beginnt.

Bevorzugt ist eine Verfahrensweise, bei der die Erhöhung der Reaktionstemperatur stufenweise erfolgt.

In einer besonders bevorzugten Ausführungsform wird dabei das Reaktionsgemisch zunächst innerhalb von 1 bis 2 Stunden auf eine Temperatur von 35 bis 50°C erhitzt und bei einem Druck von 70 bis 270 mbar das Azeotrop vollständig abdestilliert. Danach wird das Reaktionsgemisch unter Beibehaltung des obengenannten Drucks und unter vollständiger Abtrennung des Azeotrops innerhalb von 2 bis 5 Stunden auf eine Temperatur von 50 bis 70°C erhitzt. Schließlich wird dann das Reaktionsgemisch unter Beibehaltung des obengenannten Drucks und unter Abtrennung des Azeotrops innerhalb von 2 bis 5 Stunden auf eine Temperatur von 70 bis 120°C erhitzt und 5 bis 20 Stunden bei dieser Temperatur belassen. In dieser letzten Umsetzungsphase ist es besonders vorteilhaft, das Azeotrop bei einem Abnahme-/Rücklaufverhältnis von 9 : 99 bis 4 : 80 abzudestillieren.

Nach beendeter Umsetzung, die im allgemeinen 15 bis 25 Stunden in Anspruch nimmt, wird das Reaktionsgemisch abgekühlt und die Apparatur belüftet. Der Destillationsrückstand wird dann filtriert und vom Filtrat wird überschüssiges C₄-C₁₀-Alkanol abdestilliert.

C₄-C₁₀-Alkanol, das bei der azeotropen Destillation oder bei der "Nachdestillation" anfällt, kann dem Verfahren wieder zugeführt werden.

Bei den Cyanessigsäure-C₄-C₁₀-Alkylestern handelt es sich um wertvolle Zwischenprodukte für die Herstellung von Wirkstoffen, Klebstoffen oder Farbmitteln. Abhängig vom jeweiligen Verwendungszweck kann der das Zielprodukt enthaltende Destillationsrückstand direkt für weitere Syntheseschritte verwendet werden oder aber auch einer Feindestillation unterworfen werden.

Mittels des neuen Verfahrens, das sowohl kontinuierlich als auch diskontinuierlich vorgenommen werden kann, lassen sich Cyanessigsäure-C₄-C₁₀-alkylester in einfacher Weise, in guter Raum-Zeit-Ausbeute und hoher Reinheit herstellen. Ein besonderer Vorteil des neuen Verfahrens liegt darin, daß es wäßrig durchgeführt werden kann, d.h. man kann beispielsweise eine wäßrige Lösung von Cyanessigsäure, wie sie bei der Synthese der Cyanessigsäure anfällt, direkt verwenden.

Die folgenden Beispiele sollen die Erfindung näher erläutern.

### Beispiel 1

475 g 70 gew.-%ige wäßrige Cyanessigsäure, 1215 g n-Butanol und 10,25 g konz. Schwefelsäure wurden in einer Reaktionsapparatur vorgelegt. Es wurde innerhalb von 1 Stunde auf eine Innentemperatur von 42°C erwärmt und ein Druck von 70 mbar eingestellt, wobei bei vollständiger Abnahme ab 36°C das n-Butanol/Wasser-Azeotrop anfing abzudestillieren. Anschließend erhitzte man innerhalb von 3 Stunden auf 62°C und nahm bei vollständigem Ablauf und einer Übergangstemperatur von 36 bis 53°C das Azeotrop ab. Innerhalb einer Stunde wurde das Reaktionsgemisch dann weiter erhitzt (Badtemperatur: 112°C) und 8 Stunden bei dieser Temperatur gehalten, wobei bei einem Abnahme-/Rücklaufverhältnis von 5 : 80 und einer Übergangstemperatur von 46 bis 57°C das optimale Azeotrop abdestillierte und die Innentemperatur schließlich bis 90°C (Siedetemperatur von n-Butanol bei 70 mbar) stieg. Man kühlte innerhalb von 3 Stunden auf 20°C ab und belüftete die Apparatur.

Der Destillationsrückstand wurde filtriert und das Filtrat von restlichem n-Butanol mittels Destillation befreit. Man erhielt 572,7 g Rohprodukt, das einen Gehalt von 93 % (GC) Cyanessigsäure-n-butylester aufwies.

### Beispiel 2

Man verfuhr analog Beispiel 1, destillierte das Rohprodukt jedoch bei einem Druck von 1 mbar (Siedepunkt: 79-81°C). Man erhielt den Cyanessigsäure-n-butylester in einer Reinheit > 98 % (GC).

### Beispiel 3

495,2 g 68,7 gew.-%ige wäßrige Cyanessigsäure, 1215 g n-Butanol und 5 g p-Toluolsulfonsäure wurden vorgelegt. Es wurde innerhalb von 3 Stunden auf eine Innentemperatur von 60°C erhitzt und ein Druck von 72 bis 69 mbar eingestellt. Dabei ging bei vollständiger Abnahme ab 38°C das n-Butanol/Wasser-Azeotrop über. Anschließend stellte man einen Druck von 150 mbar ein und erwärmte auf 70 bis 80°C, wobei man bei einem Abnahme-/Rücklaufverhältnis von 9 : 99 16 Stunden lang das Azeotrop abnahm (Übergangstemperatur: 53 bis 60°C). Man kühlte innerhalb von 3 Stunden auf Raumtemperatur ab und belüftete die Apparatur. Der Destillationsrückstand wurde filtriert und das Filtrat von restlichem n-Butanol befreit. Das so erhaltene Rohprodukt wies einen Gehalt an Cyanessigsäure-n-butylester von 90 % (GC) auf.

### Beispiel 4

495,2 g 68,7 gew.-%ige wäßrige Cyanessigsäure, 1500 ml Isobutanol und 5 g p-Toluolsulfonsäure wurden in 3 Stunden unter einem Druck von ca. 270 mbar auf 80°C erwärmt, wobei das Wasser/Isobutanol-Azeotrop komplett abgenommen wurde. Anschließend wurde weitere 14 Stunden bei einem Abnahme-/Rücklaufverhältnis von 11 : 99 gerührt, wobei eine Übergangstemperatur von 67°C bei einer Endtemperatur von 88°C und 200 mbar erreicht wurde. Nach Filtration wurde überschüssiges Isobutanol abdestilliert und 529 g roher Cyanessigsäureisobutylester erhalten (Gehalt laut GC: 90 %).

## Patentansprüche

1. Verfahren zur Herstellung von Cyanessigsäure-C₄-C₁₀-alkylestern durch Umsetzung von Cyanessigsäure mit C₄-C₁₀-Alkanolen, dadurch gekennzeichnet, daß man Cyanessigsäure in wäßrigem Medium mit der 5 bis 30 molaren Menge, bezogen auf 1 mol Cyanessigsäure, eines C₄-C₁₀-Alkanols bei einer Temperatur von 35 bis 150°C und bei einem Druck von 70 bis 1013 mbar in Gegenwart von katalytischen Mengen einer Säure umsetzt und während der Umsetzung ein C₄-C₁₀-Alkanol/Wasser-Azeotrop abtrennt.

2. Verfahren nach Anspruch 1, dadurch gekennzeichnet, daß man Cyanessigsäure mit einem C₄-C₈-Alkanol umsetzt.

3. Verfahren nach Anspruch 1, dadurch gekennzeichnet, daß man die Umsetzung bei einer Temperatur von 50 bis 90°C vornimmt.

4. Verfahren nach Anspruch 1, dadurch gekennzeichnet, daß man die Umsetzung bei einem Druck von 70 bis 270 mbar vornimmt.

5. Verfahren nach Anspruch 1, dadurch gekennzeichnet, daß man die Umsetzung mit einer wäßrigen Cyanessigsäurelösung, die 20 bis 70 Gew.-% Cyanessigsäure, bezogen auf das Gewicht der Lösung, aufweist, vornimmt.

## Claims

1. A process for the preparation of C₄-C₁₀-alkyl cyanoacetates by reacting cyanoacetic acid with C₄-C₁₀-alkanols, which comprises reacting cyanoacetic acid with from 5 to 30 times the molar amount, based on 1 mol of cyanoacetic acid, of a C₄-C₁₀-alkanol in aqueous medium at from 35 to 150°C and at from 70 to 1013 mbar in the presence of catalytic amounts of an acid, and removing a C₄-C₁₀-alkanol/water azeotrope during the reaction.

2. A process as claimed in claim 1, wherein cyanoacetic acid is reacted with a C₄-C₈-alkanol.

3. A process as claimed in claim 1, wherein the reaction is carried out at from 50 to 90°C.

4. A process as claimed in claim 1, wherein the reaction is carried out at from 70 to 270 mbar.

5. A process as claimed in claim 1, wherein the reaction is carried out with an aqueous cyanoacetic acid solution containing from 20 to 70% by weight of cyanoacetic acid, based on the weight of the solution.

## Revendications

1. Procédé de préparation d'esters alkyliques en C₄-C₁₀ d'acide cyano-acétique par réaction d'acide cyano-acétique avec des alcanols en C₄-C₁₀, caractérisé en ce que l'on fait réagir de l'acide cyano-acétique en milieu aqueux avec la quantité 5 à 30 fois molaire, par rapport à 1 mode d'acide cyano-acétique, d'un alcanol en C₄-C₁₀, à une température de 35 à 150°C et sous une pression de 70 à 1013 mbar, en présence de quantités catalytiques d'un acide, et on sépare, pendant la réaction, un azéotrope alcanol en C₄-C₁₀/eau.

2. Procédé selon la revendication 1, caractérisé en ce que l'on fait réagir de l'acide cyano-acétique avec un alcanol en C₄-C₈.

3. Procédé selon la revendication 1, caractérisé en ce que l'on mène la réaction à une température de 50 à 90°C.

4. Procédé selon la revendication 1, caractérisé en ce que l'on mène la réaction sous une pression de 70 à 270 mbar.

5. Procédé selon la revendication 1, caractérisé en ce que l'on mène la réaction avec une solution aqueuse d'acide cyano-acétique qui contient 20 à 70% en poids d'acide cyano-acétique par rapport au poids de la solution.
